(19) Europäisches Patentamt
European Patent Office
Office européen des brevets

(11) Veröffentlichungsnummer: **0 032 879**
**A1**

(12) # EUROPÄISCHE PATENTANMELDUNG

(21) Anmeldenummer: **81810010.9**

(22) Anmeldetag: **15.01.81**

(51) Int. Cl.³: **C 07 D 277/60**, A 01 N 47/36

(30) Priorität: **21.01.80 CH 464/80**

(43) Veröffentlichungstag der Anmeldung: **29.07.81**
**Patentblatt 81/30**

(84) Benannte Vertragsstaaten: **BE CH DE FR GB IT LI NL**

(71) Anmelder: **CIBA-GEIGY AG, Patentabteilung Postfach, CH-4002 Basel (CH)**

(72) Erfinder: **Brunner, Hans-Georg, Dr., Wannenstrasse 14, CH-4415 Lausen (CH)**
Erfinder: **Föry, Werner, Dr., Benkenstrasse 65, CH-4054 Basel (CH)**

(54) **Neue Benzthiazolyl-harnstoffderivate, deren Herstellung, sie enthaltende Mittel und deren Verwendung als Herbizide.**

(57) Neue herbizid wirksame und das Pflanzenwachstum hemmende Benzthiazolylharnstoffderivate werden beschrieben. Sie entsprechen der Formel

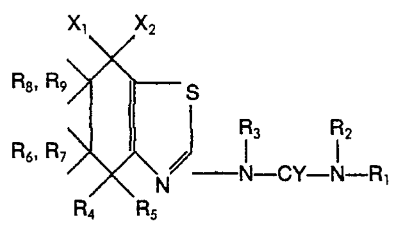

worin
$R_1$ Wasserstoff, $C_1$-$C_6$ Alkyl, $C_3$-$C_6$ Alkenyl, Alkinyl oder Cycloalkyl,
$R_2$ dasselbe wie $R_1$ oder $C_1$-$C_4$ Alkoxy,
$R_1$ und $R_2$ zusammen mit dem Stickstoffatom, an das sie gebunden sind, auch einen 5- bis 6gliedrigen Heterocyclus, der durch $C_1$-$C_3$ Alkyl substituiert sein kann,
$R_3$ beispielsweise Wasserstoff, $C_1$-$C_6$ Alkyl, ein Alkali- oder Erdalkalimetallkation oder einen $C_1$-$C_4$ Alkylammoniorest, dessen Alkylteile durch OH, $NH_2$, CN oder $C_1$-$C_4$ Alkoxy substituiert sein können,
die Reste $R_5$ bis $R_9$ bevorzugt Wasserstoff oder $C_1$-$C_4$ Alkyl, sie können jedoch auch eine der in der Beschreibung näher definierte andere Bedeutung haben,
$X_1$ und $X_2$ unabhängig voneinander je einen $C_1$-$C_8$ Alkoxy- oder Alkylthiorest, der durch Halogen substituiert und/oder durch Sauerstoff oder Schwefel unterbrochen sein kann,
$X_1$ und $X_2$ zusammen auch ein doppelt gebundenes Sauerstoffatom oder ein $C_2$-$C_3$ Alkylendioxid oder -disulfid, das im Alkylteil durch beispielsweise Halogen, $C_1$-$C_6$ Alkyl oder Alkoxy oder $C_1$-$C_4$ Alkylthio substituiert sein kann und
Y ein Sauerstoff- oder Schwefelatom bedeutet.

EP 0 032 879 A1

0032879

CIBA-GEIGY AG
Basel (Schweiz)

5-12678/=

# Neue Benzthiazolyl-harnstoffderivate, deren Herstellung, sie enthaltende Mittel und deren Verwendung als Herbizide.

Die vorliegende Erfindung betrifft neue Benzthiazolylharnstoffderivate, welche herbizide und das Pflanzenwachstum hemmende Wirkung haben. Die Erfindung umfasst ebenfalls die Herstellung dieser Verbindungen, sie enthaltende Mittel und deren Verwendung zur Bekämpfung unerwünschten Pflanzenwachstums, vor allem in Nutzpflanzenkulturen.

Aus der Literatur sind ähnlich strukturierte Benzthiazolylharnstoffe bekannt geworden, siehe z.B. die DOS 1 932 699 und die US Patentschrift No. 3 682 045. Die Benzthiazolyl-harnstoffderivate vorliegender Erfindung sind neu und zeichnen sich durch ausgezeichnete herbizide Wirkung aus.

Die neuen Benzthiazolyl-harnstoffderivate entsprechen der Formel I

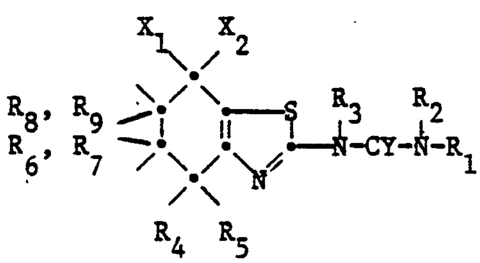

(I)

worin $R_1$ Wasserstoff, $C_1$-$C_6$ Alkyl, $C_3$-$C_6$ Alkenyl, Alkinyl oder Cycloalkyl;

$R_2$ dasselbe wie $R_1$ oder $C_1$-$C_4$ Alkoxy;

$R_1$ und $R_2$ zusammen mit dem Stickstoffatom, an das sie gebunden sind, auch einen 5-6 gliedrigen Heterocyclus, der durch $C_1$-$C_3$ Alkyl substituiert sein kann;

$R_3$ Wasserstoff, $C_1$-$C_6$ Alkyl, $C_3$-$C_6$ Alkenyl oder Alkinyl oder einen Rest $\frac{1}{n}$ $M^{n\oplus}$

M ein n-wertiges Alkali- oder Erdalkalimetallkation oder einen Ammonio-Rest

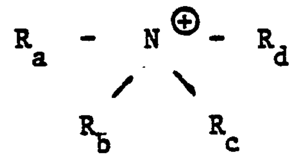

$R_a$, $R_b$, $R_c$ und $R_d$ unabhängig voneinander Wasserstoff, Benzyl oder $C_1$-$C_4$ Alkyl, das gegebenenfalls durch OH, $NH_2$, CN oder $C_1$-$C_4$ Alkoxy substituiert sein kann;

$R_4$ und $R_8$ unabhängig voneinander Wasserstoff, Halogen, $C_1$-$C_8$ Alkyl, gegebenenfalls substituiert durch Halogen oder unterbrochen durch Sauerstoff oder Schwefel;

$R_5$ und $R_9$ unabhängig voneinander dasselbe wie $R_4$, daneben Cyan, Phenyl, unsubstituiert oder substituiert durch Halogen, Nitro, Trifluormethyl, $C_1$-$C_4$ Alkoxy oder $C_1$-$C_4$ Alkylthio, die Carboxylgruppe, eine $C_1$-$C_4$ Alkoxycarbonyl- oder $C_1$-$C_4$ Alkylthiocarbonylgruppe, das $\frac{1}{n} M^{n\oplus}$ Carboxylsalz, die Carbamoylgruppe oder eine N-($C_1$-$C_8$ Alkyl)-carbamoylgruppe, deren Alkylrest durch Halogen substituiert und/oder durch Sauerstoff oder Schwefel unterbrochen sein kann;

$R_6$ und $R_7$ unabhängig voneinander Wasserstoff,

- $C_1$-$C_8$ Alkyl, gegebenenfalls halogensubstituiert oder unterbrochen durch Sauerstoff oder Schwefel,
- Phenyl unsubstituiert oder substituiert durch Halogen, Nitro, Trifluormethyl, $C_1$-$C_4$ Alkoxy oder $C_1$-$C_4$ Alkylthio;
- $C_3$-$C_8$ Cycloalkyl, gegebenenfalls substituiert durch Halogen, $C_1$-$C_6$ Alkoxy oder $C_1$-$C_4$ Alkylthio,
- $C_3$-$C_8$ Alkenyl oder Alkinyl, gegebenenfalls substituiert durch Halogen oder unterbrochen durch Sauerstoff oder Schwefel;

$R_6$ und $R_7$ können zusammen auch eine $C_2$-$C_5$ Alkylenkette bilden, die durch Halogen, $C_1$-$C_6$ Alkyl oder Alkoxy oder $C_1$-$C_4$ Alkylthio substituiert sein kann;

$R_4$ und $R_6$ sowie $R_6$ und $R_8$ können zusammen eine $C_1$-$C_5$ Alkylenkette bilden, die durch Halogen, $C_1$-$C_6$ Alkyl oder Alkoxy oder $C_1$-$C_4$ Alkylthio substituiert sein können;

Y ein Sauerstoff- oder Schwefelatom und

$X_1$ und $X_2$ unabhängig voneinander je einen $C_1$-$C_8$ Alkoxy- oder Alkylthiorest, der durch Halogen substituiert und durch Sauerstoff oder Schwefel unterbrochen sein kann,

$X_1$ und $X_2$ zusammen ein doppelt gebundenes Sauerstoffatom, ein $C_2$-$C_4$ Alkylendioxyd oder -disulfid, das im Alkylteil durch Halogen, $C_1$-$C_6$ Alkyl oder Alkoxy, $C_1$-$C_4$ Alkylthio oder Halomethyl substituiert sein kann.

In diesen Definitionen haben die Alkylreste oder die Alkylteile von Alkoxy- oder Alkylthioresten die angegebene Anzahl Kohlenstoffatome. Bevorzugt sind es jedoch niedere Reste die unverzweigt sein können,wie Methyl, Ethyl, Propyl und Butyl oder verzweigt, wie Isopropyl, sec.Butyl, Isobutyl und Tertiät Butyl. Die bevorzugten Alkenyl und Alkinylreste sind Allyl, Methally, Buten-2-yl, Propargyl, Methylpropargyl sowie Butin-2-yl. Bevorzugte Cycloalkylreste sind Cyclopropyl, Cyclopentyl und Cyclohexyl. Alkylenketten haben bevorzugt 3 bis 5 Kettenglieder, so dass mit den Kohlenstoffatomen, an die sie gebunden sind 5- und 6-Ringe entstehen. Die von $R_1$ und $R_2$ gebildeten Heterocyclen haben ebenfalls 5 oder 6 Ringglieder und sind bevorzugt Pyrol, Pyrrolidin, Pyridin, Methylpyridin, Piperidin oder Morpholin.

Die neuen Verbindungen der Formel I zeigen eine gute herbizide Wirkung auf grasartige wie auf breitblättrige Unkräuter, wobei interessante Selektivitäten festgestellt wurden bei monokotylen Kulturen, wie Mais und Getreiden, aber auch bei einzelnen dikotylen Kulturpflanzen z.B. Soja. Die neuen Verbindungen können sowohl im Vor- wie auch im Nachauflaufverfahren als Unkrautmittel eingesetzt werden. Normalerweise werden Aufwandmengen, die zwischen 0,1 und 5 kg pro Hektar liegen, benötigt.

Bei genügend grosser Aufwandmenge ist auch totalherbizide Wirkung vorhanden. Die Anwendung kann sowohl im Vorauflauf- wie im Nachauflaufverfahren vorgenommen werden. Dabei können die Aufwandmengen in weiten Grenzen schwanken, z.B. zwischen 0,1 bis 10 kg Wirkstoff pro Hektare.

Ferner besitzen die Verbindungen der Formel I günstige wachstumsregulierende Effekte (Wuchshemmung). Insbesondere hemmen sie das Wachstum der Pflanzen. Beispiele für die nutzbringende Anwendung der erfindungsgemässen Verbindungen sind z.B.

- die Reduktion des vegetativen Wachstums bei Soja und ähnlichen Leguminosen, was zu einer Ertragssteigerung dieser Kultur führt;
- die Hemmung des unerwünschten Wachstums von Geiztrieben bei Tabak, dessen Haupttrieb man geschnitten hat, was der Ausbildung grösserer und schönerer Blätter zugute kommt;
- die Hemmung des Wachstums von Gras und dikotylen Pflanzen, wie Obstbäume, Zierbäume, Gebüsche und Hecken, zwecks Einsparung an Schnittarbeit;
- die Hemmung des vegetativen Wachstums bei Getreiden, was zu Pflanzen mit kürzeren, festen Stengeln führt, die sich durch Einwirkung von Wind und Regen nicht so leicht umlegen lassen.

Die Verbindungen der Formel I können ebenfalls zum Ablösen von Blättern und Austrocknen von oberirdischen Pflanzenteilen verwendet werden, wo dies erwünscht wird. Kartoffel- und Baumwollkulturen werden beispielsweise kurz bevor deren Ernte so behandelt.

Besonders gute Wirkung zeigten diejenigen Verbindungen der Formel I, in denen

$R_1$ Wasserstoff oder $C_1$-$C_4$ Alkyl;

$R_2$ $C_1$-$C_6$ Alkyl oder $C_1$-$C_4$ Alkoxy;

$R_3$ Wasserstoff, $C_1$-$C_4$ Alkyl, ein Alkali- oder Erdalkalimetallkation, oder eine $C_1$-$C_4$ Alkylammoniogruppe, deren Alkylreste durch OH, CN oder $C_1$-$C_4$ Alkoxy substituiert sein können;
$R_4$ und $R_5$ je Wasserstoff,
$R_6$ und $R_7$ je Wasserstoff, $C_1$-$C_4$ Alkyl oder eines davon auch Phenyl,
$R_8$ und $R_9$ je Wasserstoff, $C_1$-$C_4$ Alkyl oder eines davon auch Phenyl oder einen $C_1$-$C_4$ Alkoxycarbonylrest,
$X_1$ und $X_2$ zusammen ein doppelt gebundenes Sauerstoffatom und
Y Sauerstoff oder Schwefel bedeuten.

Die besten Resultate wurden mit den Verbindungen der Formel I erzielt, in denen

$R_1$ Wasserstoff oder $C_1$-$C_6$ Alkyl,
$R_2$ $C_1$-$C_6$ Alkyl oder Methoxy,
$R_3$ Wasserstoff oder Methyl,
$R_4$ und $R_5$ je Wasserstoff,
$R_6$ und $R_7$ je Wasserstoff oder $C_1$-$C_4$ Alkyl,
$R_8$ und $R_9$ je Wasserstoff oder $C_1$-$C_4$ Alkyl,
$X_1$ und $X_2$ zusammen ein doppelt gebundenes Sauerstoffatom und
Y Sauerstoff bedeuten.

Die erfindungsgemässen Mittel enthalten ausser dem Wirkstoff der Formel I einen geeigneten Träger und/oder andere Zuschlagstoffe. Geeignete Träger und Zuschlagstoffe können fest oder flüssig sein und entsprechen den in der Formulierungstechnik üblichen Stoffen wie z.B. natürlichen oder regenerierten mineralischen Stoffen, Lösungs-, Verdünnungs-, Dispergier-, Emulgier-, Netz-, Haft-, Verdickungs-, Binde- und/oder Düngmitteln.

Zur Verwendung in herbiziden Mitteln kann die Verbindung der Formel I als Stäubemittel, Emulsionskonzentrat, als Granulat, Dispersion oder auch als Lösung oder Aufschlämmung in üblicher Formulierung verarbeitet werden.

Die Herstellung der Benzthiazolyl-harnstoffderivate der Formel I erfolgt gemäss an sich bekannter Methoden durch Umsetzen vom entsprechend substituierten Benzthiazolamin oder -isocyanat mit einem Carbamoylhalogenid, einem Isocyanat oder einem Amin.

Ein Verfahren zur Herstellung der Benzthiazolyl-harnstoffderivate der Formel I ist dadurch gekennzeichnet, dass man ein Benzthiazolamin der Formel II

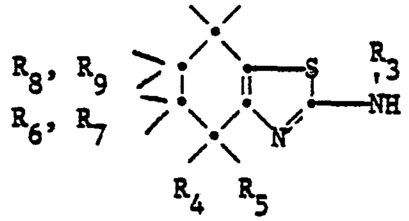

(II)

worin $R_3$ bis $R_9$ sowie $X_1$ und $X_2$ die unter Formel I gegebene Bedeutung haben, in einem inerten Lösungs- oder Verdünnungsmittel, in Anwesenheit der säurebindenden Menge einer Base, mit einem Carbamoylhalogenid der Formel III umsetzt,

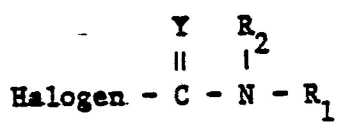

(III)

worin $R_1$, $R_2$ und Y die unter Formel I gegebene Bedeutung haben.

Ein Verfahren zur Herstellung derjenigen Benzthiazolylharnstoffderivate der Formel I, worin $R_3$ Wasserstoff bedeutet, besteht darin, dass man ein Benzthiazol-isocyanat oder -isothiocyanat der Formel IV,

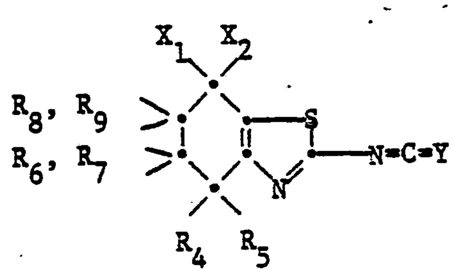

(IV)

worin $R_4$ bis $R_9$ sowie $X_1$, $X_2$ und Y die unter Formel I gegebene Bedeutung haben, in einem inerten Lösungs- oder Verdünnungsmittel mit einem Amin der Formel V umsetzt

$$H - N(R_2) - R_1 \qquad (V)$$

worin $R_1$ und $R_2$ die unter Formel I gegebene Bedeutung haben. Das Wasserstoffatom $R_3$ kann gewünschtenfalls in Gegenwart einer Base mit geeigneten Alkylestern oder mit einer starken Base durch eine Alkylgruppe oder einen basischen Salzrest ersetzt werden.

Ein Verfahren zur Herstellung der Benzthiazolyl-harnstoffderivate der Formel I, worin $R_2$ Wasserstoff bedeutet, besteht darin, dass man ein Benzthiazolamin der Formel II in einem inerten Lösungs- oder Verdünnungsmittel mit einem Isocyanat oder Isothiocyanat der Formel VI umsetzt,

$$R_1 - N = C = Y \qquad (VI)$$

worin $R_1$ und Y die unter Formel I gegebene Bedeutung haben. Das Wasserstoffatom $R_2$ kann gewünschtenfalls in Gegenwart einer Base mit einem geeigneten Alkylester durch einen Alkylrest ausgetauscht werden.

Diese Umsetzungen werden vorteilhafterweise in protischen, organischen Lösungs- oder Verdünnungsmitteln durchgeführt wie z.B. Alkoholen, Ester, Aethern, Ketonen, Dimethylformamid, Dimethylsulfoxyd, Acetonitril, allein oder verdünnt mit Wasser.

Die Reaktionstemperaturen liegen zwischen -10° und + 150°C, praktisch aber zwischen der Raumtemperatur und dem Siedepunkt des Lösungsmittelgemisches. Die Reaktionsdauer beträgt je nach dem gewählten Ausgangsstoffen, dem Lösungsmittel und der Temperatur 1 Stunde bis ca. einen Tag.

Wo ein Halogenatom bei der Reaktion abgespaltet wird, sollte die aquimolare Menge eines säurebindenden Mittels eingesetzt werden. Als solches eignet sich im Prinzip jede anorganische oder organische Base wie z.B. NaOH, KOH, $NaHCO_3$, $K_2CO_3$ oder K-tert. Butylat. Bevorzugt werden jedoch sekundäre und tertiäre Amine wie Trimethylamin, Triäthylamin, Pyridin, 4-Dimethylamino-pyridin,K-tert.Butylat etc.

Die als Ausgangsstoffe benützten Benzthiazolamine der Formel II werden erhalten durch Umsetzten von entsprechend substituierten Cyclohexan-1,3-dionen mit Thioharnstoffen in einem alkanolischen Lösungsmittel in Gegenwart von etwas Jod als Katalysator z.B. gemäss folgendem Schema.

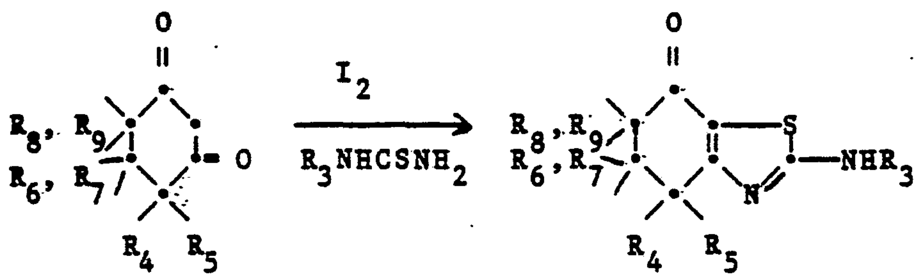


VII II

Ferner können die Benzthiazol-amine der Formel II auch durch Kondensieren von Aminonitril in Gegenwart von Schwefel an ein 3-Oxo-4,5-dihydro-6H-anilin, gemäss dem Schema

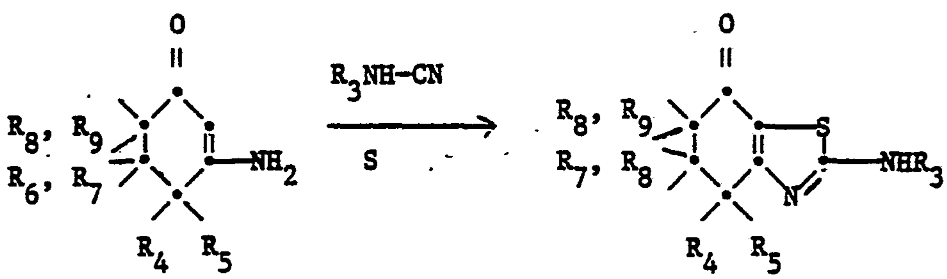


erhalten werden.

Die Dione der Formel VII sowie die Aniline der Formel VIII können ebenfalls mittels eines Aminoiso-thiocyanates der Formel IX

$$R_3NH — N = C = S \qquad (IX)$$

zum Ringschluss gebracht werden. In diesen Formeln haben $R_1$ bis $R_9$ die unter Formel I gegebene Bedeutung.
Solche Kondensationen sind in Weissenberger, "The Chemistry of heterocyclic compounds"

Vol 34 part 1, p. 213 ff, part 2, p. 9 ff, sowie
Vol 39 part 1, p. 297 ff (John Wiley + Sons 1979)

nachgesehen werden. Ueber die Herstellung der Ausgangsmaterialien oder ähnlicher Produkte enthalten ebenfalls die DOS 1 932 699, die US Patente 3 682 045 und 4 120 690 sowie die schweizerische Patentschrift No. 528 533 Angaben.

Die neuen Wirkstoffe der Formel I sind stabile Verbindungen, welche in üblichen organischen Lösungsmitteln, wie Alkoholen, Aethern, Ketonen, Dimethylformamid, Dimethylsulfoxid etc. löslich sind. Sie sind nicht explosiv oder ätzend und ihre Handhabung bedarf keiner besonderer Vorsichtsmassnahmen.

Die Herstellung erfindungsgemässer Mittel erfolgt in an sich bekannter Weise durch inniges Vermischen und Vermahlen von Wirkstoffen der Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden:

Feste Aufarbeitungsformen: Stäubemittel, Streumittel, Granulate, Umhüllungsgranulate, Imprägnierungsgranulate und Homogengranulate;

In Wasser dispergierbare Wirkstoffkonzentrate:

Spritzpulver (wettable powder), Pasten, Emulsionen, Emulsionspräparate;

Flüssige Aufarbeitungsformen: Lösungen, Dispersionen.

Die Wirkstoffkonzentrationen betragen in den erfindungsgemässen Mitteln 1 bis 80 Gewichtsprozent und können gegebenenfalls bei der Anwendung auch in geringen Konzentrationen wie etwa 0,05 bis 1% vorliegen.

Den beschriebenen erfindungsgemässen Mitteln lassen sich andere biozide Wirkstoffe oder Mittel beimischen. So können die neuen Mittel ausser den genannten Verbindungen der Formel I z.B. Insektizide, Fungizide, Bakterizide, Fungistatika, Bakteriostatika, Nematozide oder weitere Herbizide zur Verbreiterung des Wirkungsspektrums enthalten.

Die folgenden Beispiele beschreiben im Detail die Herstellung einiger erfindungsgemässer Benzthiazolyl-harnstoffderivate der Formel I und sie enthaltende Mittel. Weitere in analoger Weise erhaltene Wirkstoffe sind in der sich dem Beispiel 5 anschliessenden Tabelle aufgeführt. Die Temperaturen sind in Centigraden angegeben, Druckangaben in Millibar während sich Teile und Prozentangaben auf das Gewicht beziehen.

Beispiel 1: N-(5,5-Dimethyl-7-oxo-4,5-dihydro-6,H-benzthiazol-2-yl)-N'-methyl-harnstoff

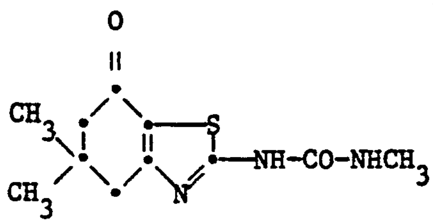

a) 28 g 2-Brom-5,5-dimethyl-cylohexan-1,3-dion und 11,7 g Thioharnstoff werden in 200 ml Ethanol gelöst. Nach dem Abklingen der schwach exothernen Reaktion wurde noch 6 Stunden am Rückfluss gekocht. Die Lösung wurde mit 400 ml Wasser verdünnt und mit gesättigter Natriumbicarbonatlösung neutralisiert. Das Kristallisat wurde abfiltriert und am Vakuum bis 80° getrocknet. Man erhielt 21 g 2-Amino-5,5-dimethyl-7-oxo-4,5-dihydro-6 H-benzthiazol vom Schmelzpunkt 203-205°.

b) 9 g des gemäss a) erhaltenen Produktes und 4 g Methylisocyanat wurden in 150 ml Acetonitril gelöst und über Nacht bei Raumtemperatur gerührt. Die Lösung wurde auf $\frac{1}{3}$ des Volumens eingeengt und dann auf 0° gekühlt. Die ausgefallenen Kristalle wurden abfiltriert und im Vakuum bei 80° getrocknet. Man erhielt so 11,5 g der Titelverbindung als farblose Kristalle mit Schmelzpunkt 228-231° (Verbindung 1)

Beispiel 2: N-(5,5-Dimethyl-7-oxo-4,5-dihydro-6H-benzthiazol-2-yl)-N'-methyl-N'-methoxy-harnstoff

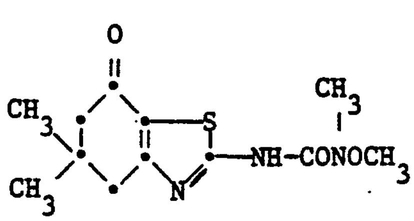

15 g 2-Amino-5,5-dimethyl-7-oxo-4,5-dihydro-6H-benzthiazol (erhalten gemäss Beispiel 1a) wurden in 100 ml Dimethylformamid gelöst und 8,6 g

Kalium-tert.-Butylat zugegeben. Anschliessend wurden bei 0° 11.3 g Methoxymethylcarbamoylchlorid zugetropft und 3 Stunden bei Raumtemperatur gerührt. Dann wurde das Gemisch auf Eiswasser gegossen und mit Chloroform extrahiert. Nach einer Chromatographie an Kieselgel mit Chloroform: Methanol 20:1 erhielt man 8 g der Titelverbindung vom Schmelzpunkt 145-148° (Verbindung 3).

Beispiel 3: N-(5,5-Dimethyl-7-oxo-4,5-dihydro-6H-benzthiazol-2-yl)-N',N'-dimethyl-harnstoff

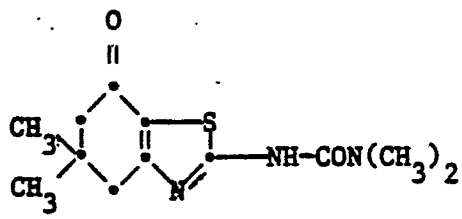

Analog Beispiel 2 wurden aus 12g 2-Amino-5,5-dimethyl-7-oxo-4,5-dihydro-6H-benzthiazol (Beispiel 1a), 7,2 g Kalium-tert.Butylat und 8,6 g Dimethylcarbamoylchlorid, 7,6 g der Titelverbindung vom Schmelzpunkt 175-180° erhalten (Verbindung 4).

Beispiel 4: N-Methyl-N-(5,5-dimethyl-7-oxo-4,5-dihydro-6H-benzthiazol-2-yl)-N'-methyl-harnstoff

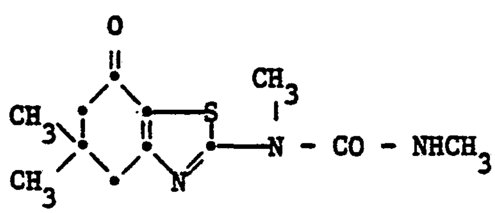

a) 82 g 2-Brom-5,5-dimethyl-cyclohexan-1,3-dion, 40 g Na-acetat und 50 g N-methyl-thioharnstoff wurden in 500 ml Essigsäure gelöst. Anschliessend wurde bei Raumtemperatur gerührt und bis die schwach exotherme Reaktion abgeklungen war, darauf noch 3 Stunden am Rückfluss gekocht. Das Reaktionsgemisch wurde am Rotationsverdampfer auf $\frac{1}{3}$ des Volumens eingeengt, dann auf 100 ml Wasser gegossen und auf 0° gekühlt. Das Produkt wurde abfiltriert und im Vakuum bei 80° getrocknet. Es wurden so 64 g 2-Methylamino-5,5-dimethyl-7-oxo-4,5-dihydro-6H-benzthiazol erhalten mit Schmelzpunkt 217-218°.

b) 15 g des unter a) erhaltenen Produktes und 5 ml Methylisocyanat wurden in 100 ml Acetonitril suspendiert und über Nacht bei Raumtemperatur gerührt. Als Katalysator wurde 1 ml Trimethylamin zugefügt. (In einem anderen Versuch wurde mit gleichem Erfolg 100 mg 4-Dimethylaminopyridin zugefügt). Die Reaktionslösung wurde dann am Rotationsverdampfer eingeengt und aus einem Gemisch von Chloroform: Hexan 1:1 kristallisiert. Man erhielt so 14 g der Titelverbindung mit Schmelzpunkt 174-175° (Verbindung 4).

<u>Beispiel 5:</u> N-Methyl-N-(5,5-dimethyl-7-oxo-4,5-dihydro-6H-benzthiazol-2-yl)-N'-tert.butyl-harnstoff

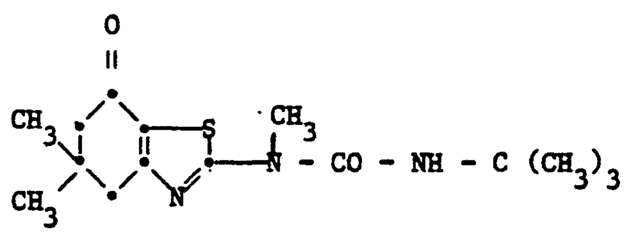

15 g 2-Methylamino-5,5-dimethyl-7-oxo-4,5-dihydro-6H-benzthiazol (erhalten im Beispiel 4a) wurden in 100 ml Acetonitril suspendiert. Bei 0° wurden dann 8,2 g tert.Butylisocyanat und 1 g Kalium-tert. Butylat zugegeben. Anschliessend wurde 24 Stunden bei Raumtemperatur gerührt. Nach dem Aufarbeiten, welches wie im Beispiel 4b beschrieben erfolgte, erhielt man 10,8 g der Titelverbindung als farblose Kristalle mit Schmelzpunkt 165-167° (Verbindung 9).

Analog zu diesen Beispielen wurden weitere Verbindungen erhalten:

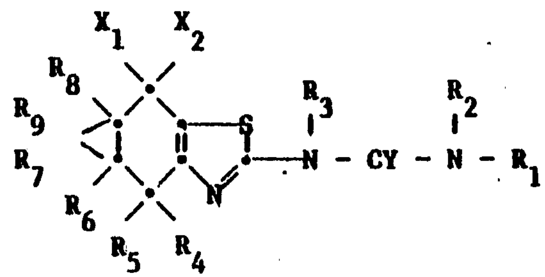

| NO. | $R_8$, $R_9$ | $R_6$, $R_7$ | $R_4$, $R_5$ | $X_1$, $X_2$ | Y | $R_3$ | $NR_1R_2$ | phys. Daten |
|---|---|---|---|---|---|---|---|---|
| 1 | $H_2$ | $(CH_3)_2$ | $H_2$ | O | O | H | $NHCH_3$ | Smp 228-231° |
| 2 | $H_2$ | $(CH_3)_2$ | $H_2$ | O | O | $CH_3$ | $NHCH_3$ | Smp 174-175° |
| 3 | $H_2$ | $(CH_3)_2$ | $H_2$ | O | O | H | $N(CH_3)OCH_3$ | Smp 145-8° |
| 4 | $H_2$ | $(CH_3)_2$ | $H_2$ | O | O | H | $N(CH_3)_2$ | Smp 175-180° |
| 5 | $H_2$ | $(CH_3)_2$ | $H_2$ | O | O | $CH_3$ | $N(CH_3)OCH_3$ | Smp 108-110° |
| 6 | $H_2$ | $(CH_3)_2$ | $H_2$ | O | O | H | $NHC_4H_9n$ | Smp 204-6° |
| 7 | $H_2$ | $(CH_3)_2$ | $H_2$ | O | O | $CH_3$ | $NHC_4H_9n$ | Smp 124-6° |
| 8 | $H_2$ | $(CH_3)_2$ | $H_2$ | O | O | $CH_3$ | $NHC_4H_9$ tert | Smp 165-7° |
| 9 | $H_2$ | $(CH_3)_2$ | $H_2$ | O | O | $CH_3$ | $N(CH_3)_2$ | Smp 165-7° |
| 10 | $H_2$ | $(CH_3)_2$ | $H_2$ | O | O | Na | $N(CH_3)_2$ | |
| 11 | $H_2$ | $(CH_3)_2$ | $H_2$ | O | O | Na | $N(CH_3)$ $OCH_3$ | |
| 12 | $H_2$ | $(CH_3)_2$ | $H_2$ | O | S | $CH_3$ | $NHCH_3$ | Smp 157-60° |
| 13 | $H_2$ | $(CH_3)_2$ | $H_2$ | O | O | $NH_3C_3H_7$iso | $N(CH_3)OCH_3$ | |

| NO. | $R_8$, $R_9$ | $R_6$, $R_7$ | $R_4$, $R_5$ | $X_1$, $X_2$ | Y | $R_3$ | $NR_1R_2$ | phys. Daten |
|---|---|---|---|---|---|---|---|---|
| 14 | $H_2$ | $(CH_3)_2$ | $H_2$ | 0 | 0 | $NH_3C_3H_7$ iso | $N(CH_3)\ OCH_3$ | |
| 15 | $H_2$ | $(CH_3)_2$ | $H_2$ | 0 | 0 | $NH(CH_3)_3$ | $N(CH_3)OCH_3$ | |
| 16 | $H_2$ | $(CH_3)_2$ | $H_2$ | 0 | 0 | H | 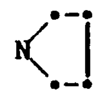 | |
| 17 | $H_2$ | $(CH_3)_2$ | $H_2$ | 0 | 0 | $NH(CH_3)_3$ | $N(CH_3)_2$ | |
| 18 | $H_2$ | $(CH_3)_2$ | $H_2$ | 0 | 0 | H | 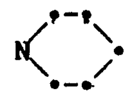 | |
| 19 | $H_2$ | $(CH_3)_2$ | $H_2$ | 0 | 0 | H | 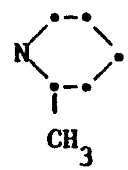 | |
| 20 | $H_2$ | $(CH_3)_2$ | H, $COOCH_3$ | 0 | 0 | $CH_3$ | $NHCH_3$ | |
| 21 | $H_2$ | $(CH_3)_2$ | H, $COOCH_3$ | 0 | 0 | $CH_3$ | $NHC_4H_9$ n | |
| 22 | $H_2$ | $(CH_3)_2$ | H, $COOCH_3$ | 0 | 0 | H | $N(CH_3)_2$ | |
| 23 | $H_2$ | $(CH_3)_2$ | H, $COOCH_3$ | 0 | 0 | H | $N(CH_3)OCH_3$ | |
| 24 | $H_2$ | $(CH_3)_2$ | H, $COOCH_3$ | 0 | S | $CH_3$ | $NHCH_3$ | |
| 25 | $H_2$ | $(CH_3)_2$ | H,CN | 0 | 0 | $CH_3$ | $NHCH_3$ | |
| 26 | $H_2$ | $(CH_3)_2$ | H,CN | 0 | 0 | $CH_3$ | $N(CH_3)OCH_3$ | |

| No. | $R_8$, $R_9$ | $R_6$, $R_7$ | $R_4$, $R_5$ | $X_1$, $X_2$ | Y | $R_3$ | $NR_1R_2$ | phys. Daten |
|---|---|---|---|---|---|---|---|---|
| 27 | $H_2$ | $(CH_3)_2$ | H,CN | 0 | 0 | H | $N(CH_3)_2$ | |
| 28 | $H_2$ | $(CH_3)_2$ | H, 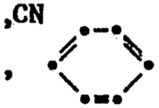 | 0 | 0 | $CH_3$ | $NHCH_3$ | |
| 29 | $H_2$ | $(CH_3)_2$ | H, 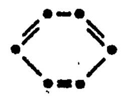 | 0 | 0 | H | $N(CH_3)_2$ | |
| 30 | $H_2$ | $(CH_3)_2$ | H, 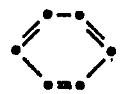 | 0 | 0 | H | $N(CH_3)OCH_3$ | |
| 31 | $H_2$ | $(CH_3)_2$ | $CH_3$, $COOCH_3$ | 0 | 0 | $CH_3$ | $NHCH_3$ | |
| 32 | $H_2$ | $(CH_3)_2$ | $CH_3$, $COOCH_3$ | 0 | 0 | $CH_3$ | $NHC_4H_9$ n | |
| 33 | $H_2$ | $(CH_3)_2$ | $CH_3$, $COOCH_3$ | 0 | 0 | H | $N(CH_3)_2$ | |
| 34 | $H_2$ | $(CH_3)_2$ | $CH_3$, $COOCH_3$ | 0 | 0 | H | $N(CH_3)$ $OCH_3$ | |
| 35 | $H_2$ | $(CH_3)_2$ | $CH_3$, COOH | 0 | 0 | $CH_3$ | $NHCH_3$ | |
| 36 | $H_2$ | $(CH_3)_2$ | $CH_3$, COOH | 0 | 0 | H | $N(CH_3)_2$ | |
| 37 | $H_2$ | $(CH_3)_2$ | $CH_3$, COOH | 0 | 0 | H | $N(CH_3)OCH_3$ | |
| 38 | $H_2$ | $(CH_3)_2$ | H, Br | 0 | 0 | H | $N(CH_3)_2$ | |
| 39 | $H_2$ | $(CH_3)_2$ | H,Br | 0 | 0 | $CH_3$ | $NHCH_3$ | |
| 40 | $H_2$ | $(CH_3)_2$ | H, Br | 0 | 0 | H | $N(CH_3)OCH_3$ | |
| 41 | $H_2$ | $(CH_3)_2$ | H,$COOCH_3$ | 0 | 0 | Na | $N(CH_3)_2$ | |
| 42 | $H_2$ | $(CH_3)_2$ | H,$COOCH_3$ | 0 | 0 | Na | $N(CH_3)OCH_3$ | |

| No. | $R_8$, $R_9$ | $R_6$, $R_7$ | $R_4$, $R_5$ | $X_1$, $X_2$ | Y | $R_3$ | $NR_1R_2$ | phys. Daten |
|---|---|---|---|---|---|---|---|---|
| 43 | $H_2$ | $(CH_3)_2$ | H, $COOCH_3$ | O | O | $NH_3C_3H_7$ iso | $N(CH_3)_2$ | |
| 44 | $H_2$ | $(CH_3)_2$ | H, $COOCH_3$ | O | O | $NH_3C_3H_7$ iso | $N(CH_3)OCH_3$ | |
| 45 | $H_2$ | $(CH_3)_2$ | H, $COOCH_3$ | O | O | $NH(CH_3)_3$ | $N(CH_3)_2$ | |
| 46 | $H_2$ | $(CH_3)_2$ | H, $COOCH_3$ | O | O | $NH(CH_3)_3$ | $N(CH_3)OCH_3$ | |
| 47 | $H_2$ | $(CH_3)_2$ | H, $COOCH_3$ | O | O | H | 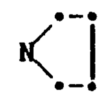 | |
| 48 | $H_2$ | $(CH_3)_2$ | H, $COOCH_3$ | O | O | H | 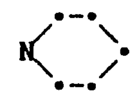 | |
| 49 | $H_2$ | $(CH_3)_2$ | H, $COOCH_3$ | O | O | H | 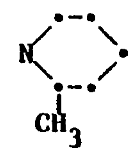 | |
| 50 | $H_2$ | $(CH_3)_2$ | H, $CONH_2$ | O | O | H | $N(CH_3)_2$ | |
| 51 | $H_2$ | $(CH_3)_2$ | H, $CONHC_2H_5$ | O | O | H | $N(CH_3)_2$ | |
| 52 | $H_2$ | $(CH_3)_2$ | H, $COOC_4H_9n$ | O | O | H | $N(CH_3)_2$ | |
| 53 | H, $COOCH_3$ | $(CH_3)_2$ | $H_2$ | O | O | $CH_3$ | $NHCH_3$ | Smp.152-154° |
| 54 | H, $COOCH_3$ | $(CH_3)_2$ | $H_2$ | O | O | $CH_3$ | $NHC_4H_9n$ | |
| 55 | H, $COOCH_3$ | $(CH_3)_2$ | $H_2$ | O | O | H | $N(CH_3)_2$ | |
| 56 | H, $COOCH_3$ | $(CH_3)_2$ | $H_2$ | O | O | H | $N(CH_3)OCH_3$ | Smp 80-88° |
| 57 | H, $COOCH_3$ | $(CH_3)_2$ | $H_2$ | O | S | $CH_3$ | $NHCH_3$ | |

| No. | $R_8,R_9$ | $R_6,R_7$ | $R_4,R_5$ | $X_1,X_2$ | Y | $R_3$ | $NR_1R_2$ | phys. Daten |
|---|---|---|---|---|---|---|---|---|
| 58 | H, $CH_3$ | $(CH_3)_2$ | $H_2$ | 0 | 0 | $CH_3$ | $NHCH_3$ | Smp. 133-136° |
| 59 | H, CN | $(CH_3)_2$ | $H_2$ | 0 | 0 | H | $N(CH_3)_2$ | |
| 60 | H, $CH_3$ | $(CH_3)_2$ | $H_2$ | 0 | 0 | $CH_3$ | $N(CH_3)OCH_3$ | Smp. 111-118° |
| 61 | H, 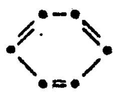 | $(CH_3)_2$ | $H_2$ | 0 | 0 | $CH_3$ | $NHCH_3$ | |
| 62 | H, 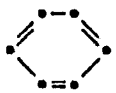 | $(CH_3)_2$ | $H_2$ | 0 | 0 | H | $N(CH_3)_2$ | |
| 63 | H, 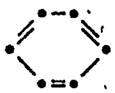 | $(CH_3)_2$ | $H_2$ | 0 | 0 | H | $N(CH_3)_2OCH_3$ | |
| 64 | $CH_3$,$COOCH_3$ | $(CH_3)_2$ | $H_2$ | 0 | 0 | $CH_3$ | $NHCH_3$ | Smp. 178-179° |
| 65 | $CH_3$,$COOCH_3$ | $(CH_3)_2$ | $H_2$ | 0 | 0 | $CH_3$ | $NHC_4H_9$ n | |
| 66 | $CH_3$,$COOCH_3$ | $(CH_3)_2$ | $H_2$ | 0 | 0 | H | $N(CH_3)_2$ | |
| 67 | $CH_3$,$COOCH_3$ | $(CH_3)_2$ | $H_2$ | 0 | 0 | H | $N(CH_3)OCH_3$ | Smp. 100-104° |
| 68 | $CH_3$,COOH | $(CH_3)_2$ | $H_2$ | 0 | 0 | $CH_3$ | $NHCH_3$ | |
| 69 | $CH_3$,COOH | $(CH_3)_2$ | $H_2$ | 0 | 0 | H | $N(CH_3)_2$ | |
| 70 | $CH_3$,COOH | $(CH_3)_2$ | $H_2$ | 0 | 0 | H | $N(CH_3)OCH_3$ | |
| 71 | Br,H | $(CH_3)_2$ | $H_2$ | 0 | 0 | H | $N(CH_3)_2$ | |
| 72 | Br,H | $(CH_3)_2$ | $H_2$ | 0 | 0 | $CH_3$ | $NHCH_3$ | |
| 73 | Br,H | $(CH_3)_2$ | $H_2$ | 0 | 0 | H | $N(CH_3)OCH_3$ | |

| No. | $R_8$, $R_9$ | $R_6$, $R_7$ | $R_4$, $R_5$ | $X_1$, $X_2$ | Y | $R_3$ | $NR_1R_2$ | phys. Daten |
|---|---|---|---|---|---|---|---|---|
| 74. | H,$COOCH_3$ | $(CH_3)_2$ | $H_2$ | O | O | Na | $N(CH_3)_2$ | |
| 75 | H,$COOCH_3$ | $(CH_3)_2$ | $H_2$ | O | O | Na | $N(CH_3)OCH_3$ | |
| 76 | H,$COOCH_3$ | $(CH_3)_2$ | $H_2$ | O | O | $NH_3C_3H_7$ iso | $N(CH_3)_2$ | |
| 77 | H,$COOCH_3$ | $(CH_3)_2$ | $H_2$ | O | O | $NH_3C_3H_7$ iso | $N(CH_3)OCH_3$ | |
| 78 | H,$COOCH_3$ | $(CH_3)_2$ | $H_2$ | O | O | $NH(CH_3)_3$ | $N(CH_3)_2$ | |
| 79 | $H_2$ | $C_3H_7$iso,H | $H_2$ | O | O | $CH_3$ | $N(CH_3)OCH_3$ | Smp. 106-107° |
| 80 | H,$COOCH_3$ | $(CH_3)_2$ | $H_2$ | O | O | H | 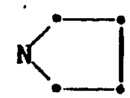 | |
| 81 | $H_2COOCH_3$ | $(CH_3)_2$ | $H_2$ | O | O | H | 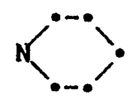 | |
| 82 | H,$COOCH_3$ | $(CH_3)_2$ | $H_2$ | O | O | H | 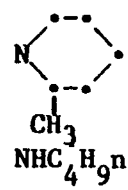 | |
| 83 | H | $C_4H_9$(t),H | $H_2$ | O | O | $CH_3$ | $NHC_4H_9n$ | Smp. 125-129° |
| 84 | H, $CH_3$ | $(CH_3)_2$ | $H_2$ | O | O | $CH_3$ | $NHC_4H_9n$ | Smp. 92- 95° |
| 85 | $H_2$ | $(CH_3)_2$ | $H_2$ | O | O | $CH_3$ | $NHC_2H_5$ | Smp. 144-145° |
| 86 | $H_2$ | $H_2$ | $H_2$ | O | O | H | $N(CH_3)_2$ | Smp 178-181° |
| 87 | $H_2$ | $H_2$ | $H_2$ | O | O | H | $NHCH_3$ | Smp 225-228° |
| 88 | $H_2$ | $H_2$ | $H_2$ | O | O | H | $N(CH_3)OCH_3$ | Smp 165-167° |
| 89 | $H_2$ | $H_2$ | $H_2$ | O | O | $CH_3$ | $NHCH_3$ | |

| No. | $R_8$,$R_9$ | $R_6$,$R_7$ | $R_4$, $R_5$ | $X_1$,$X_2$ | Y | $R_3$ | $NR_1R_2$ | phys. Daten |
|---|---|---|---|---|---|---|---|---|
| 90 | $H_2$ | $H_2$ | H, 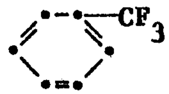 | O | O | H | $N(CH_3)_2$ | |
| 91 | $H_2$ | $H_2$ | H, 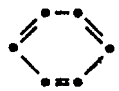 | O | O | H | $N(CH_3)OCH_3$ | |
| 92 | $H_2$ | | H, 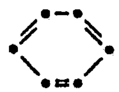 | O | O | $CH_3$ | $NHCH_3$ | |
| 93 | H, 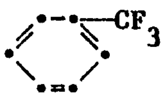 | $H_2$ | $H_2$ | O | O | H | $N(CH_3)_2$ | |
| 94 | H, 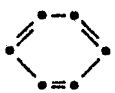 | $H_2$ | $H_2$ | O | O | H | $N(CH_3)OCH_3$ | |
| 95 | H, 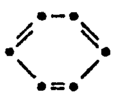 | $H_2$ | $H_2$ | O | O | $CH_3$ | $NHCH_3$ | |
| 96 | $H_2$ | H, 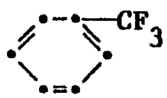 | $H_2$ | O | O | H | $N(CH_3)_2$ | |
| 97 | $H_2$ | H, 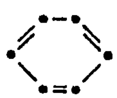 | $H_2$ | O | O | H | $N(CH_3)OCH_3$ | |
| 98 | $H_2$ | H, 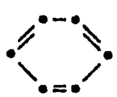 | $H_2$ | O | O | $CH_3$ | $NHCH_3$ | Smp. 170-175° |
| 99 | $H_2$ | H, 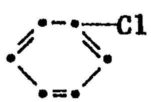 | H,$COOCH_3$ | O | O | H | $N(CH_3)_2$ | |

| No. | $R_8,R_9$ | $R_6,R_7$ | $R_4,R_5$ | $X_1,X_2$ | Y | $R_3$ | $NR_1R_2$ | phys. Daten |
|---|---|---|---|---|---|---|---|---|
| 100 | $H_2$ | H, 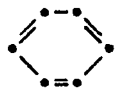 | $H,COOCH_3$ | O | O | H | $N(CH_3)OCH_3$ | |
| 101 | $H_2$ | H, 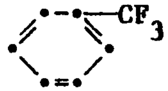 | $H,COOCH_3$ | O | O | $CH_3$ | $NHCH_3$ | |
| 102 | $H,COOCH_3$ | H, 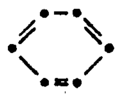 | $H_2$ | O | O | H | $N(CH_3)_2$ | Smp. 140-150° |
| 103 | $H,COOCH_3$ | H, 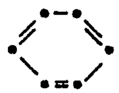 | $H_2$ | O | O | H | $N(CH_3)OCH_3$ | Smp. 80° |
| 104 | $H,COOCH_3$ | H, 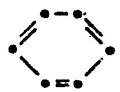 | $H_2$ | O | O | $CH_3$ | $NHCH_3$ | Smp. 173-175°C |
| 105 | $H_2$ | $H,C_3H_7$ iso | $H_2$ | O | O | H | $N(CH_3)_2$ | |
| 106 | $H_2$ | $H,C_3H_7$ iso | $H_2$ | O | O | H | $N(CH_3)OCH_3$ | |
| 107 | $H_2$ | $H,C_3H_7$ iso | $H_2$ | O | O | $CH_3$ | $NHCH_3$ | Smp. 134-136° |
| 108 | $H_2$ | $H,C_3H_7$ iso | $H,COOCH_3$ | O | O | H | $N(CH_3)_2$ | |
| 109 | $H_2$ | $H,C_3H_7$ iso | $H,COOCH_3$ | O | O | H | $N(CH_3)OCH_3$ | |
| 110 | $H_2$ | $H,C_3H_7$ iso | $H,COOCH_3$ | O | O | $CH_3$ | $NHCH_3$ | |
| 111 | $H,COOCH_3$ | $H,C_3H_7$ iso | $H_2$ | O | O | H | $N(CH_3)_2$ | |
| 112 | $H,COOCH_3$ | $H,C_3H_7$ iso | $H_2$ | O | O | H | $N(CH_3)OCH_3$ | Smp. 185-190° |
| 113 | $H,COOCH_3$ | $H,C_3H_7$ iso | $H_2$ | O | O | $CH_3$ | $NHCH_3$ | Smp. 130-135° |
| 114 | $H_2$ | $H,C_4H_9t$ | $H_2$ | O | O | H | $N(CH_3)_2$ | |

| No. | $R_8$, $R_9$ | $R_6$, $R_7$ | $R_4$, $R_5$ | $X_1$,$X_2$ | Y | $R_3$ | $NR_1R_2$ | phys. Daten |
|---|---|---|---|---|---|---|---|---|
| 115 | $H_2$ | $H,C_4H_9t$ | $H_2$ | 0 | 0 | H | $N(CH_3)OCH_3$ | Smp. 95° |
| 116 | $H_2$ | $H,C_4H_9t$ | $H_2$ | 0 | 0 | $CH_3$ | $NHCH_3$ | Smp. 150-152° |
| 117 | $H_2$ | $H,C_4H_9t$ | $H,COOCH_3$ | 0 | 0 | H | $N(CH_3)_2$ | |
| 118 | $H_2$ | $H,C_4H_9t$ | $H,COOCH_3$ | 0 | 0 | H | $N(CH_3OCH_3$ | |
| 119 | $H_2$ | $H,C_4H_9t$ | $H,COOCH_3$ | 0 | 0 | $CH_3$ | $NHCH_3$ | |
| 120 | $H,COOCH_3$ | $H,C_4H_9$ t | $H_2$ | 0 | 0 | H | $N(CH_3)_2$ | |
| 121 | $H,COOCH_3$ | $H,C_4H_9t$ | $H_2$ | 0 | 0 | H | $N(CH_3)OCH_3$ | |
| 122 | $H,COOCH_3$ | $H,C_4H_9t$ | $H_2$ | 0 | 0 | $CH_3$ | $NHCH_3$ | |
| 123 | $H_2$ | $H,C=CH$ 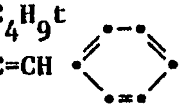 | $H_2$ | 0 | 0 | $CH_3$ | $NHCH_3$ | |
| 124 | $H_2$ | H, 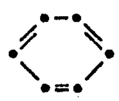 | H, 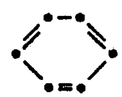 | 0 | 0 | H | $N(CH_3)_2$ | |
| 125 | $H_2$ | $H,CH=CH-CH_3$ | $H_2$ | 0 | 0 | $CH_3$ | $NHCH_3$ | |
| 126 | $H_2$ | $H,CH_2CH(CH_3)$<br>$S-C_2H_5$ | $H_2$ | 0 | 0 | $CH_3$ | $NHCH_3$ | |
| 127 | H, 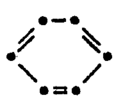 | H, 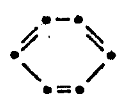 | $H_2$ | 0 | 0 | $CH_3$ | $NHCH_3$ | |
| 128 | H, $(CH_2)_4$ | H | $H_2$ | 0 | 0 | $CH_3$ | $NHCH_3$ | |
| 129 | H $(CH_2)_4$ | H | $H_2$ | 0 | 0 | $CH_3$ | $NHCH_3$ | |
| 130 | $H_2$ | $(CH_3)_2$ | $H_2$ | $O(CH_2)_2O$ | 0 | $CH_3$ | $NHCH_3$ | |

| No. | $R_8$, $R_9$ | $R_6$, $R_7$ | $R_4$, $R_5$ | $X_1$, $X_2$ | Y | $R_3$ | $NR_1R_2$ | phys. Daten |
|---|---|---|---|---|---|---|---|---|
| 131 | $H_2$ | $(CH_3)_2$ | $H_2$ | $S(CH_2)_2S$ | O | $CH_3$ | $NHCH_3$ | |
| 132 | $H_2$ | $(CH_3)_2$ | $H_2$ | $O(CH_2)_2O$ | O | H | $N(CH_3)_2$ | |
| 133 | $H_2$ | $(CH_3)_2$ | H, $COOCH_3$ | $O(CH_2)_2O$ | O | H | $N(CH_3)_2$ | |
| 134 | $H_2$ | $H_2$ | $H_2$ | $O(CH_2)_2O$ | O | $CH_3$ | $NHCH_3$ | |
| 135 | H, $COOCH_3$ | $(CH_3)_2$ | $H_2$ | $O(CH_2)_2O$ | O | $CH_3$ | $NHCH_3$ | |
| 136 | $H_2$ | H, $C_3H_7$ iso | $CH_3$ $COOCH_3$ | $O(CH_2)_2O$ | O | H | $N(CH_3)OCH_3$ | |
| 137 | H, $CH_3$ | H, $C_3H_7$ (i) | H | O | O | $CH_3$ | $N(CH_3)_2$ | |
| 138 | H, $CH_3$ | H, $C_3H_7$(i) | $H_2$ | O | O | $CH_3$ | $NHCH_3$ | |
| 139 | H, $C_2H_5$ | H, $C_3H_7$(i) | $H_2$ | O | O | $CH_3$ | $NHC_4H_9$(n) | |
| 140 | H, $C_2H_5$ | $(CH_3)_2$ | $H_2$ | O | O | $CH_3$ | $N(CH_3)_2$ | |
| 141 | H $C_2H_5$ | $(CH_3)_2$ | $H_2$ | O | O | $CH_3$ | $NHCH_3$ | |
| 142 | H $C_2H_5$ | $C_3H_7$(i), H | $H_2$ | O | O | H | $N(CH_3)OCH_3$ | |
| 143 | H $C_3H_7$n | $(CH_3)_2$ | $H_2$ | O | O | $CH_3$ | $NHCH_3$ | |
| 144 | H $C_3H_7$n | $(CH_3)_2$ | $H_2$ | O | O | H | $N(CH_3)_2$ | |
| 145 | H $C_4H_9$n | $(CH_3)_2$ | $H_2$ | O | O | $CH_3$ | $NHCH_3$ | |
| 146 | H $C_4H_9$n | $(CH_3)_2$ | $H_2$ | O | O | H | $N(CH_3)OCH_3$ | |

Beispiel 6:

Die Aufarbeitung der Wirkstoffe der Formel I zu herbiziden Mitteln kann z.B. gemäss einer der nachstehenden Formulierungsbeispiele erfolgen.

Granulat:

Zur Herstellung eines 5%igen Granulates werden die folgenden Zutaten verwendet:

5 Teile N-(5,5-Dimethyl-7-oxo-4,5-dihydro-6H-benzthiazol-2-yl)-N'-methyl-harnstoff,
0,25 Teile Epichlorhydrin,
0,25 Teile Cetylpolyglykoläther,
3,50 Teile Polyäthylenglykol,
91 Teile Kaolin (Korngrösse: 0,3 - 0,8 mm).

Die Aktivsubstanz wird mit Epichlorhydrin vermischt und in 6 Teilen Aceton gelöst, hierauf wird Polyäthylenglykol und Cetylpolyglykoläther zugesetzt. Die so erhaltene Lösung wird auf Kaolin aufgesprüht und anschliessend im Vacuum verdampft.

Spritzpulver

Zur Herstellung eines a) 70%igen und b) 10%igen Spritzpulvers werden folgende Bestandteile verwendet:

a) 70 Teile N-(5,5-Dimethyl-7-oxo-4,5-dihydro-6H-benzthiazol-2-yl)-N'-methyl-N'-methoxy-harnstoff
5 Teile Natriumdibutylnaphtylsulfonat,
3 Teile Naphthalinsulfonsäuren-Phenolsulfonsäuren-Formaldehyd-Kondensat 3:2:1,
10 Teile Kaolin,
12 Teile Champagne-Kreide;

b) 10 Teile des obigen Wirkstoffes,
3 Teile Gemisch der Natriumsalze von gesättigten Fettalkoholsulfaten,

5 Teile Naphthalinsulfonsäuren-Formaldehyd-Kondensat,
82 Teile Kaolin.

Der Wirkstoff wird auf die entsprechenden Trägerstoffe (Kaolin und Kreide) aufgezogen und anschliessend vermischt und vermahlen. Man erhält Spritzpulver von vorzüglicher Benetzbarkeit und Schwebefähigkeit. Aus solchen Spritzpulvern können durch Verdünnung mit Wasser Suspension von 0,1 - 8% Wirkstoff erhalten werden, die sich zur Unkrautbekämpfung in Pflanzenkulturen eignen.

Paste

Zur Herstellung einer 45%igen Paste werden folgende Stoffe verwendet:

45 Teile N-(5,5-Dimethyl-7-oxo-4,5-dihydro-6H-benzthioazol-2-yl)-N',N'-dimethyl-harnstoff
5 Teile Natriumaluminiumsilikat,
14 Teile Cetylpolyglykoläther mit 8 Mol Aethylenoxid,
1 Teil Oleylpolyglykoläther mit 5 Mol Aethylenoxid,
2 Teile Spindeloel
10 Teile Polyäthylenglykol,
23 Teile Wasser.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Emulsionskonzentrat

Zur Herstellung eines 25%igen Emulsionskonzentrates werden

25 Teile N-Methyl-N-(5,5-dimethyl-7-oxo-4,5-dihydro-6H-benzthiazol-2-yl)-N'-methyl-harnstoff
5 Teile einer Mischung von Monylphenolpolyoxyäthylen oder Calciumdodecylbenzolsulfat,

15 Teile Cyclohexanon,
55 Teile Xylol

miteinander vermischt. Dieses Konzentrat kann mit Wasser zu Emulsionen auf geeignete Konzentrationen verdünnt werden.

Beispiel 7:

Zum Nachweis der herbiziden und pflanzenwuchshemmenden Wirkung wurden die Verbindungen der Formel I Versuchen gemäss folgenden Testmethoden unterworfen.

Pre-emergente Herbizidwirkung (Vorauflaufwirkung)

Im Gewächshaus werden Pflanzensamen in Blumentöpfe von 12-15 cm Durchmesser gesät, so dass sich pro Topf 10-25 Pflänzchen entwickeln können. Unmittelbar nach der Einsaat wird die Erdoberfläche mit einer wässrigen Suspension der Wirkstoffe, erhalten aus einem 10%igen Spritzpulver, behandelt. Es werden vier verschiedene Konzentrationsreihen angewendet, entsprechend 4, 2, 1 und 0,5 kg Wirksubstanz pro Hektar. Die Blumentöpfe werden im Gewächshaus bei 22-25°C und 50-70% relativer Luftfeuchtigkeit und regelmässigem Begiessen gehalten. Der Versuch wird nach 3 Wochen ausgewertet und der Zustand der Pflanzen gemäss folgendem Bonitierungsschema beurteilt:

9 = Pflanze gedeiht normal, wie unbehandelte Kontrollpflanze
8-2 = zunehmende Stufen der Schädigung
1 = Pflanze abgestorben.

Die Resultate sind in der untenstehenden Tabelle zusammengefasst.

| Verbindung No. | 2 | | 3 | | 4 | | 9 | | 58 | |
|---|---|---|---|---|---|---|---|---|---|---|
| Aufwandmenge kg/ha: | 4 | 2 | 4 | 2 | 4 | 2 | 4 | 2 | 4 | 2 |
| Pflanze: | | | | | | | | | | |
| Avena fatua | 1 | 1 | 1 | 2 | 1 | 1 | 1 | 1 | 1 | 1 |
| Bromus tectorum | 1 | 1 | 2 | 3 | 1 | 2 | 1 | 1 | 1 | 1 |
| Lolium perenne | 1 | 1 | 2 | 2 | 2 | 3 | 1 | 1 | 1 | 2 |
| Alopecurus myos. | 1 | 1 | 2 | 2 | 2 | 2 | 1 | 1 | 1 | 1 |
| Sorghum halepense | 1 | 1 | 1 | 1 | 1 | 1 | 1 | 1 | 1 | 2 |
| Abutilon | 1 | 1 | 1 | 1 | 1 | 1 | 1 | 1 | 1 | 1 |
| Sida spinosa | 1 | 1 | 1 | 1 | 1 | 2 | 1 | 1 | 1 | 1 |
| Amaranthus retrofl. | 1 | 1 | 1 | 1 | 1 | 1 | 1 | 1 | 1 | 1 |
| Solanum nirgrum | 1 | 1 | 1 | 1 | 1 | 1 | 1 | 1 | 1 | 1 |
| Ipomoea purpurea | 1 | 1 | 1 | 1 | 1 | 1 | 1 | 1 | 1 | 1 |
| Sinapis alba | 1 | 1 | 1 | 1 | 1 | 1 | 1 | 1 | 1 | 1 |
| Stellaria media | 1 | 1 | 1 | 1 | 1 | 1 | 1 | 1 | 1 | 1 |
| Chryanthemum leuc. | 1 | 1 | 1 | 1 | 1 | 1 | 1 | 1 | 1 | 1 |

Herbizide Wirkung bei Applikation der Wirkstoffe nach dem Auflaufen der Pflanzen (post-emergent).

Verschiedene Kulturpflanzen und Unkräuter werden aus den Samen in Töpfen im Gewächshaus aufgezogen, bis sie das 4- bis 6-Blatt-Stadium erreicht haben. Dann werden die Pflanzen mit wässerigen Wirkstoffemulsionen (erhalten aus dem 5%igen Emulsionskonzentrat) in verschiedenen Dosierungen gespritzt. Die behandelten Pflanzen werden dann bei optimalen Bedingungen von Licht, regelmässigem Begiessen, 22-25°C Temperatur und 50-70%iger relativer Luftfeuchtigkeit gehalten. 15 Tage nach der Behandlung erfolgte die Auswertung des Versuches. Der Zustand der Pflanzen wurde gemäss der oben gegebenen Notenskala beurteilt, die Resultate sind in der unten stehenden Tabelle zusammengefasst.

| Verbindung No. | 2 | 3 | 4 | 5 | 9 | 58 |
|---|---|---|---|---|---|---|
| Aufwandmenge kg/ha: | 4 2 | 4 2 | 4 2 | 4 2 | 4 2 | 4 2 |
| **Pflanze:** | | | | | | |
| Avena fatua | 1 1 | 1 2 | 2 2 | 1 2 | 1 1 | 1 2 |
| Lolium perenne | 1 1 | 1 2 | 4 7 | 2 2 | 1 1 | 1 4 |
| Alopecurus myos uroides | 1 1 | 1 2 | 2 2 | 2 3 | 1 1 | 1 2 |
| Digitaria sangiunalis | 1 1 | 2 2 | 1 1 | 1 2 | 1 1 | 3 4 |
| Echinochloa crus galli | 1 1 | 2 3 | 2 2 | 2 4 | 1 2 | 1 2 |
| Abutilon | 1 1 | 1 1 | 1 1 | 1 1 | 1 1 | 1 1 |
| Sida spinosa | 1 1 | 1 1 | 1 1 | 1 1 | 1 1 | 1 1 |
| Amaranthus retrofl. | 1 1 | 1 1 | 1 1 | 1 1 | 1 1 | 1 1 |
| Solanum nigrum | 1 1 | 1 1 | 1 1 | 1 1 | 1 1 | 1 1 |
| Ipomea purpurea | 1 1 | 1 1 | 1 1 | 1 1 | 1 1 | 1 1 |
| Sinapis alba | 1 1 | 1 1 | 1 1 | 1 1 | 1 1 | 1 1 |
| Stellaria media | 1 1 | 1 1 | 1 1 | 1 1 | 1 1 | 1 1 |
| Chrysanthemum leuc. | 1 1 | 1 1 | 1 1 | 1 1 | 1 1 | 1 1 |
| Galium aparine | 1 1 | 1 1 | 1 1 | 1 1 | 1 1 | 1 1 |
| Sesbania exaltata | 1 1 | 1 1 | 1 1 | 1 1 | 1 1 | 1 1 |

Wuchsregulierung und Ertragssteigerung an Sojabohnen

In einer Klimakammer im Gewächshaus wurden Sojabohnen der Sorte "Hark" in Kunststoffbehältern in ein Erde-Torf-Sand-Gemisch (6:3:1) angesät. Durch optimale Kontrolle der Temperatur, Bewässerung und Beleuchtung sowie durch Düngergabe konnten sich nach ca. 5 Wochen Pflänzchen im 5-6 Trifola-Blattstadium entwickeln. Zu diesem Zeitpunkt wurden die Pflänzchen mit wässrigen Brühen eines Wirkstoffes in Konzentrationen von 10, 50, 100 und 500 ppm bis zur guten Benetzung (Beginn des Abtropfens) besprüht. Die Pflanzen wurden dann weitergezogen, bis 5 Wochen nach der Behandlung der Versuch ausgewertet wurde.

Gegenüber unbehandelten Kontrollpflanzen zeigten je 10 mit Verbindung No. 83 behandelte Sojapflanzen bei Aufwandmengen von 50 und 100 ppm
- eine Erhöhung des Gewichtes der abgeernteten Schoten um 13 resp. 17%
- eine Verminderung der Wuchshöhe von 9 resp. 14%
- eine durchschnittliche Verlängerung der Seitentriebbildung von 5-11%.

Dessikations- und Defoliationswirkung

In einem Gewächshaus wurden Baumwollpflanzen der Sorte Deltapine in Tontöpfen angezogen. Nach abgeschlossener Kapselbildung wurden sie mit wässrigen Zubereitungen der Wirkstoffe Nr. 1 und 2 in einer Aufwandmenge, die 1, 2, 0,6 und 0,3 kg/ha im Feld entspräche, gespritzt. Unbehandelte Pflanzen wurden als Kontrolle belassen. Die Auswertung des Versuches erfolgte 3, 7 und 14 Tage nach Applikation der Wirksubstanz durch Bestimmen des Grades an Defoliation (% abgefallene Blätter) und an Dessikation (% Austrocknung der an der Pflanze verbleibenden Blätter). Die Verbindungen Nr. 9, 58, 60 und 86 liessen nach 2 Wochen bloss noch wenige vertrocknete Blätter an den Pflanzen bei Aufwandmengen von 1,2 und 0,6 kg/ha.

Patentansprüche

1. Neue Benzthiazolyl-harnstoffderivate der Formel I

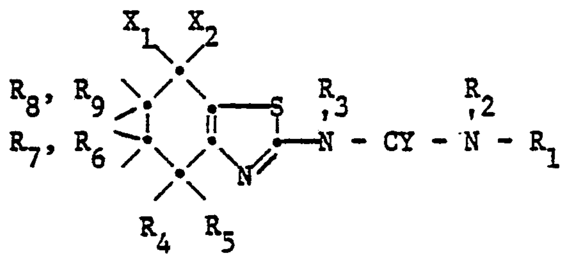

(I)

worin $R_1$ Wasserstoff, $C_1$-$C_6$ Alkyl, $C_3$-$C_6$ Alkenyl, Alkinyl oder Cycloalkyl;

$R_2$ dasselbe wie $R_1$ oder $C_1$-$C_4$ Alkoxy;

$R_1$ und $R_2$ zusammen mit dem Stickstoffatom an das sie gebunden sind, auch einen 5-6 gliedrigen Heterocyclus, der durch $C_1$-$C_3$ Alkyl substituiert sein kann;

$R_3$ Wasserstoff, $C_1$-$C_6$ Alkyl, $C_3$-$C_6$ Alkenyl oder Alkinyl oder einen Rest $\frac{1}{n} M^{n \oplus}$

M ein n-wertiges Alkali- oder Erdalkalimetallkation oder einen Ammonio-Rest

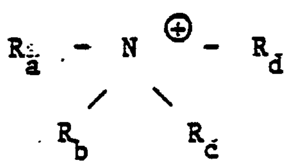

$R_a$, $R_b$, $R_c$ und $R_d$ unabhängig voneinander Wasserstoff, Benzyl oder $C_1$-$C_4$ Alkyl, das gegebenenfalls durch OH, $NH_2$, CN oder $C_1$-$C_4$ Alkoxy substituiert sein kann;

$R_4$ und $R_8$ unabhängig voneinander Wasserstoff, Halogen, $C_1$-$C_8$ Alkyl, gegebenenfalls substituiert durch Halogen oder unterbrochen durch Sauerstoff oder Schwefel;

$R_5$ und $R_9$ unabhängig voneinander dasselbe wie $R_4$, daneben Cyan, Phenyl, unsubstituiert oder substituiert durch Halogen, Nitro, Trifluormethyl, $C_1$-$C_4$ Alkoxy oder $C_1$-$C_4$ Alkylthio, die Carboxyl-

gruppe, eine $C_1$-$C_4$ Alkoxycarbonyl- oder $C_1$-$C_4$ Alkylthiocarbonylgruppe, das $\frac{1}{n} M^{n\oplus}$ Carboxylsalz, die Carbamoylgruppe oder eine N-($C_1$-$C_8$ Alkyl)-carbamoylgruppe, deren Alkylrest durch Halogen substituiert und/oder durch Sauerstoff oder Schwefel unterbrochen sein kann;

$R_6$ und $R_7$ unabhängig voneinander Wasserstoff,

- $C_1$-$C_8$ Alkyl, gegebenefalls halogensubstituiert oder unterbrochen durch Sauerstoff oder Schwefel,
- Phenyl unsubstituiert oder substituiert durch Halogen, Nitro, Trifluormethyl, $C_1$-$C_4$ Alkoxy oder $C_1$-$C_4$ Alkylthio;
- $C_3$-$C_8$ Cycloalkyl, gegebenenfalls substituiert durch Halogen, $C_1$-$C_6$ Alkoxy oder $C_1$-$C_4$ Alkylthio;
- $C_3$-$C_8$ Alkenyl oder Alkinyl, gegebenenfalls substituiert durch Halogen oder unterbrochen durch Sauerstoff oder Schwefel;

$R_6$ und $R_7$ können zusammen auch eine $C_2$-$C_5$ Alkenylkette bilden, die durch Halogen, $C_1$-$C_6$ Alkyl oder Alkoxy oder $C_1$-$C_4$ Alkylthio substituiert sein kann;

$R_4$ und $R_6$ sowie $R_6$ und $R_8$ können zusammen eine $C_1$-$C_5$ Alkylenkette bilden, die durch Halogen, $C_1$-$C_6$ Alkyl oder Alkoxy oder $C_1$-$C_4$ Alkylthio substituiert sein können;

Y ein Sauerstoff- oder Schwefelatom und

$X_1$ und $X_2$ unabhängig voneinander je einen $C_1$-$C_8$ Alkoxy- oder Alkylthiorest, der durch Halogen substituiert und durch Sauerstoff oder Schwefel unterbrochen sein kann,

$X_1$ und $X_2$ zusammen ein doppelt gebundenes Sauerstoffatom, ein $C_2$-$C_3$ Alkylendioxyd oder -disulfid, das im Alkylteil durch Halogen, $C_1$-$C_6$ Alkyl oder Alkoxy, $C_1$-$C_4$ Alkylthio oder Halomethyl substituiert sein kann.

2. Die Benzthiadiazolyl-harnstoffderivate der Formel I, Anspruch 1, worin

$R_1$ Wasserstoff oder $C_1$-$C_6$ Alkyl,

$R_2$ $C_1$-$C_6$ Alkyl oder $C_1$-$C_4$ Alkoxy,

$R_3$ Wasserstoff, $C_1$-$C_6$ Alkyl, ein Alkali- oder Erdalkalimetall-kation oder eine $C_1$-$C_4$ Alkylammoniogruppe, deren Alkylreste durch OH, CN oder $C_1$-$C_4$ Alkoxy substituiert sein können,

$R_4$ und $R_5$ je Wasserstoff,

$R_6$ und $R_7$ je Wasserstoff, $C_1$-$C_4$ Alkyl oder eines davon

$R_8$ und $R_9$ je Wasserstoff, $C_1$-$C_4$ Alkyl oder eines davon auch Phenyl oder einen $C_1$-$C_4$ Alkoxycarbonylrest,

$X_1$ und $X_2$ zusammen ein doppelt gebundenes Sauerstoffatom und

Y Sauerstoff oder Schwefel bedeuten.

3. Die Benzthiadiazolyl-harnstoffderivate der Formel I, Anspruch 1, worin

$R_1$ Wasserstoff oder $C_1$-$C_6$ Alkyl,

$R_2$ $C_1$-$C_6$ Alkyl oder Methoxy,

$R_3$ Wasserstoff oder Methyl,

$R_4$ und $R_5$ je Wasserstoff,

$R_6$ und $R_7$ je Wasserstoff oder $C_1$-$C_4$ Alkyl,

$R_8$ und $R_9$ je Wasserstoff oder $C_1$-$C_4$ Alkyl,

$X_1$ und $X_2$ zusammen ein doppelt gebundenes Sauerstoffatom und

Y Sauerstoff bedeuten.

4. N-Methyl-N-(5,5-dimethyl-7-oxo-4,5-dihydro-6H-benzthiazol-2-yl)-N',N'-dimethyl-harnstoff.

5. N-Methyl-N-(5,5-dimethyl-7-oxo-4,5-dihydro-6H-benzthiazol-2-yl)-N'-methyl-N'-methoxy-harnstoff.

6. N-Methyl-N-(5,5-dimethyl-7-oxo-4,5-dihydro-6H-benzthiazol-2-yl)-N',N'-dimethyl-harnstoff.

7. N-Methyl-N-(5,5-dimethyl-7-oxo-4,5-dihydro-6H-benzthiazol-2-yl)-N'-methyl-N'-methoxy-harnstoff.

8. N-Methyl-N-(5,5-dimethyl-7-oxo-4,5-dihydro-6H-benzthiazol-2-yl)-N'-methyl-harnstoff.

9. N-Methyl-N-(5,5,6-trimethyl-7-oxo-4,5-dihydro-6H-benzthiazol-2-yl)-N'-methyl-harnstoff.

10. N-Methyl-N-(5-tert.butyl-7-oxo-4,5-dihydro-6H-benzthiazol-2-yl)-N'-n-butyl-harnstoff.

11. N-Methyl-N-(5,5,6-trimethyl-7-oxo-4,5-dihydro-6H-benzthiazol-2-yl)-N'-methyl-N'-methoxy-harnstoff.

12. Verfahren zur Herstellung der Benzthiazolyl-harnstoffderivate der Formel I, Anspruch 1, dadurch gekennzeichnet, dass man ein Benzthiazolamin der Formel II,

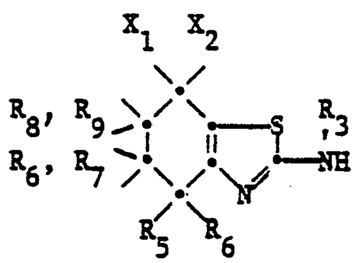

worin $R_3$ bis $R_9$ sowie $X_1$ und $X_2$ die unter Formel I im Anspruch 1 gegebene Bedeutung haben, in einem Lösungs- oder Verdünnungsmittel, in Anwesenheit der säurebindenden Menge einer Base, mit einem Carbamoylhalogenid der Formel III umsetzt,

$$\text{Halogen} - \overset{\overset{\displaystyle Y}{\|}}{C} - \overset{\overset{\displaystyle R_2}{|}}{N} - R_1 \qquad \text{(III)}$$

worin $R_1$, $R_2$ und Y die unter Formel I im Anspruch 1 gegebene Bedeutung haben.

13. Verfahren zur Herstellung der Benzthiazolyl-harnstoffderivate der Formel I, Anspruch 1, dadurch gekennzeichnet, dass man ein Benzthiazol-isocyanat oder- isothiocyanat der Formel IV,

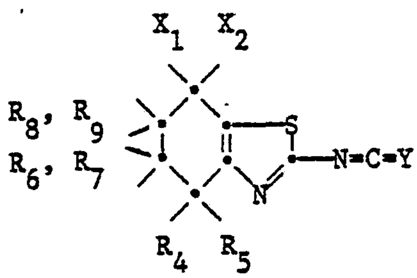

(IV)

worin $R_4$ bis $R_9$, $X_1$, $X_2$ und Y die unter Formel I im Anspruch 1 gegebene Bedeutung haben, in einem inerten Lösungs- oder Verdünnungsmittel mit einem Amin der Formel V umsetzt,

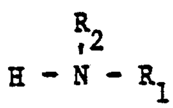

(V)

worin $R_1$ und $R_2$ die unter Formel I im Anspruch 1 gegebene Bedeutung haben und gewünschtenfalls das Wasserstoffatom $R_3$ mit einem geeigneten Alkylester, in Gegenwart einer Base durch eine Alkylgruppe ersetzt oder mit einer starken Base durch einen basischen Salzrest ersetzt.

14. Verfahren zur Herstellung der Benzthiazolyl-harnstoffderivate der Formel I, Anspruch 1, dadurch gekennzeichnet, dass man ein Benzthiazolamin der Formel II

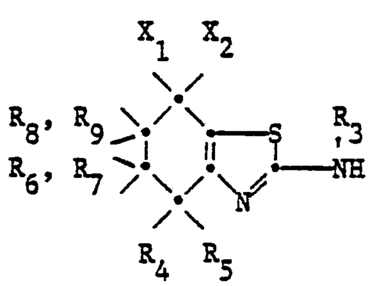

(II)

worin $R_3$ bis $R_9$ sowie $X_1$ und $X_2$ die unter Formel I im Anspruch 1 gegebene Bedeutung haben, in einem inerten Lösungs- und Verdünnungsmittel mit einem Isocyanat oder Isothiocyanat der Formel VI umsetzt,

$$R_1 - N = C = Y \qquad (VI)$$

worin $R_1$ und Y die unter Formel I im Anspruch 1 gegebene Bedeutung haben und gewünschtenfalls das Wasserstoffatom $R_2$ in Gegenwart einer Base mit einem geeigneten Alkylester durch einen Alkylrest ersetzt.

15. Herbizides und das Pflanzenwachstum hemmendes Mittel, dadurch gekennzeichnet, dass es als Wirkstoff mindestens ein Benzthiazolylharnstoffderivat der Formel I, Anspruch 1 enthält.

16. Die Verwendung der Benzthiazolyl-harnstoffderivate der Formel I, Anspruch 1 zur Bekämpfung von Unkräutern.

17. Die Verwendung der Benzthiazolyl-harnstoffderivate der Formel I, Anspruch 1 als selektive Unkrautmittel in Kulturpflanzungen.

18. Die Verwendung der Benzthiazolyl-harnstoffderivate der Formel I, im Anspruch 1 zur Hemmung des Pflanzenwachstums.

19. Die Verwendung der Benzthiazolyl-harnstoffderivate der Formel I, Anspruch 1 zum Entblättern und Austrocknen von Baumwoll- und Kartoffelpflanzen kurz bevor deren Ernte.

Europäisches Patentamt

# EUROPÄISCHER RECHERCHENBERICHT

0032879

Nummer der Anmeldung

EP 81810010.9

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | AT - B - 318 969 (BAYER)<br>+ Seiten 2-4; Ansprüche +<br>-- | 1,14-18 |
| | AT - B - 295 922 (CIBA-GEIGY)<br>+ Seiten 4-6; Ansprüche 1,3, 4,6 +<br>-- | 1,12-14 |
| | DE - A1 - 2 612 541 (ELI LILLY)<br>+ Seiten 9,10; Ansprüche +<br>-- | 1,13,14 |
| | DE - A1 - 2 548 898 (NIPPON SODA)<br>+ Seiten 2-4; Ansprüche +<br>---- | 1,12, 14-18 |

KLASSIFIKATION DER ANMELDUNG (Int. Cl.[3])

C 07 D 277/60
A 01 N 47/36

RECHERCHIERTE SACHGEBIETE (Int. Cl.[3])

C 07 D 277/00
A 01 N 43/00
A 01 N 47/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 23-03-1981 | TENGLER |